Europäisches Patentamt

European Patent Office

Office européen des brevets

(19)

(11) Numéro de publication: **0 300 901**
**A2**

# (12) DEMANDE DE BREVET EUROPEEN

(21) Numéro de dépôt: **88401880.5**

(51) Int. Cl.⁴: **G 01 N 33/14**

(22) Date de dépôt: **20.07.88**

(30) Priorité: **20.07.87 FR 8710350**

(43) Date de publication de la demande:
**25.01.89 Bulletin 89/04**

(84) Etats contractants désignés:
**AT BE CH DE ES FR GB GR IT LI LU NL SE**

(71) Demandeur: **ASSOCIATION POUR LA PROMOTION DE LA RECHERCHE ET DE L'INNOVATION TECHNOLOGIQUE (APRIT)**
**12 rue des Micocouliers**
**F-66680 Canohes (FR)**

**SICAREX-MIDI ROUSSILLON**
**Maison de l'Agriculture 9 Avenue de Grande Bretagne**
**F-66025 Perpignan Cédex (FR)**

(72) Inventeur: **Balat, Marianne**
**IMP CNRS Université de Perpignan**
**F-66025 Perpignan Cedex (FR)**

**Boiret, Michel**
**IMP CNRS Université de Perpignan**
**F-66025 Perpignan Cedex (FR)**

**Marty, Alain**
**IMP CNRS Université de Perpignan**
**F-66025 Perpignan Cedex (FR)**

**Prosdocimi, Patrick**
**IMP CNRS Université de Perpignan**
**F-66025 Perpignan Cedex (FR)**

**Spinner, Bernard**
**IMP CNRS Université de Perpignan**
**F-66025 Perpignan Cedex (FR)**

**Guittard, Alain**
**Sicarex-Midi Roussillon Alenya**
**F-66200 Elne (FR)**

(74) Mandataire: **Bouju, André**
**Cabinet Bouju 38 avenue de la Grande Armée**
**F-75017 Paris (FR)**

(54) **Procédé pour évaluer le risque de précipitation d'un composé solide dans le vin.**

(57) Le procédé pour évaluer le risque de précipitation d'un composé solide dans du vin, comprend les étapes suivantes :
- on prélève $n$ échantillons de vin,
- on porte ces échantillons à une température ($T_1$) supérieure ou égale à la température ambiante et on ajoute à ceux-ci des masses différentes dudit composé solide, de façon à obtenir un gradient de concentration connu en ce composé,
- après dissolution du composé, la température des échantillons est abaissée jusqu'à une certaine valeur ($T_2$) et est stabilisée à celle-ci,
- la température est ensuite remontée jusqu'à une valeur ($T_m$) inférieure à ($T_1$) à laquelle est observée la présence ou l'absence d'un trouble.

EP 0 300 901 A2

**Description**

## "Procédé pour évaluer le risque de précipitation d'un composé solide dans le vin"

La présente invention concerne un procédé pour évaluer le risque de précipitation d'un composé solide dans du vin.

L'ion potassium et l'acide tartrique contenus dans le raisin se retrouvent naturellement dans le vin et peuvent précipiter sous forme d'hydrogénotartrate de potassium (THK) lorsque la température du vin diminue. Une bouteille présentant un tel précipité est perçue par le client comme impropre à la consommation. Aussi, pour éviter tout risque de dépôt de THK, la quasi totalité des vins sont soumis, avant la mise en bouteille, à un traitement de stabilisation tartrique préventif.

Ce traitement est onéreux et contraignant. Or, parmi tous les vins traités sans discernement, certains présentent une stabilité naturelle tout à fait satisfaisante. D'importantes économies pourraient donc être réalisées si le traitement était réservé aux seuls vins instables.

Devant l'importante économique du problème, de nombreuses méthodes de mesure de stabilité tartrique des vins ont déjà été proposées. Toutefois, aucune de ces méthodes ne donne entière satisfaction car celles qui sont fiables pour un cru donné ne sont pas transposables à d'autres crus. Seule la méthode classique de conservation au froid reste considérée comme ayant valeur de référence : un vin peut être réputé stable si après un séjour de 6 jours à - 5°C il ne présente aucun trouble de précipitation tartrique. Cette méthode a l'inconvénient d'un temps de réponse trop long, incompatible avec les contraintes d'une installation d'embouteillage industrielle.

La présente invention a pour but de définir et de mesurer un Indice de Stabilité Tartrique (IST) et un Indice de Stabilité Tartrique Critique (ISTC) dont la connaissance permettrait de rationnaliser l'application du traitement de stabilisation tartrique en le réservant aux seuls vins réellement instables.

La présente invention a pour objet un procédé de laboratoire permettant d'attribuer un IST à un vin et un ISTC à un cru ou catégorie de vins.

Suivant l'invention, le procédé pour évaluer le risque de précipitation d'un composé solide dans du vin est caractérisé par les étapes suivantes :
- on prélève $n$ échantillons de vin,
- on porte ces échantillons à une température $(T_1)$ supérieure ou égale à la température ambiante et on ajoute à ceux-ci des masses différentes dudit composé solide, de façon à obtenir un gradient de concentration connu en ce composé,
- après dissolution du composé, la température des échantillons est abaissée jusqu'à une certaine valeur $(T_2)$ et est stabilisée à celle-ci,
- la température est ensuite remontée jusqu'à une valeur $(T_m)$ inférieure à $(T_1)$ à laquelle est observée la présence ou l'absence d'un trouble,
- on note la concentration à laquelle et au-dessous de laquelle on observe l'absence du trouble et la concentration à laquelle et au-dessus de laquelle on observe la présence d'un trouble et on caractérise l'indice de stabilité du vin testé au moyen de ces deux concentrations.

Le procédé conforme à l'invention est destiné plus particulièrement à l'évaluation du risque de précipitation d'hydrogénate de potassium (THK) ou de tartrate alcalin ou alcalino-terreux. A cet effet, on ajoute aux échantillons de vin de l'hydrogénate de potassium et/ou un tartrate alcalin ou alcalino-terreux, en quantités connues.

Le procédé selon l'invention peut cependant s'appliquer à tout composé solide susceptible de précipiter dans le vin.

Selon une version préférée de l'invention, le vin testé est préalablement filtré au moyen d'un filtre dont la porosité est comprise entre $0,1\mu m$ et $100\mu m$.

De préférence également des masses connues de composé comprises entre 0 et 1,5 g par litre sont dissoutes dans le vin testé de sorte que soit obtenue une série de n échantillons (tel que $n \geq 1$) présentant un gradient de concentration déterminé.

De manière avantageuse, la série d'échantillons est soumise à un traitement thermique comportant trois niveaux de température successifs $T_1$, $T_2$ et $T_m$ tels que $50°C > T_1 > T_m \geq T_2 > 10°C$ où $T_1$ est la température de dissolution du composé (THK), $T_2$ la température à laquelle la série d'échantillons est maintenue pendant une durée comprise entre 0 et 10 heures et $T_m$ la température à laquelle est observé le résultat du test.

Ainsi, le vin à tester est filtré à un niveau compatible avec celui des filtrations industrielles classiques et n échantillons d'un volume identique sont prélevés et placés dans une enceinte thermostatée où ils sont agités pendant la durée du test.

Trois niveaux de température sont successivement utilisés : $T_1$, $T_2$ et $T_m$ tels que $T_1 > T_m \geq T_2$. A la température de $T_1$ des masses différentes de THK solide sont ajoutées et dissoutes de sorte que la série des n échantillons présente un gradient de concentration connu en THK dissout. Après dissolution totale, la température est abaissée et stabilisée à $T_2$ pendant une durée H, puis éventuellement remontée à $T_m$. Cette température $T_m$ est celle à laquelle est observée la présence ou l'absence d'un trouble tartrique par observation visuelle directe ou par mesure physicochimique (conductimétrie, turbidimétrie).

Deux concentrations sont alors remarquables : celle à laquelle et au-dessous de laquelle on observe la présence d'un trouble tartrique, celle à laquelle et au-dessus de laquelle on observe la présence d'un trouble tartrique. Ces deux concentrations exprimées en mg de THK ajouté à 100 ml de vin caractérisent l'IST du vin testé. L'écart entre les deux concentrations traduit la précision du test.

L'exemple suivant donné à titre indicatif et non limitatif illustre l'invention.

Le vin dont on veut déterminer l'IST est filtré sur membrane de porosité égale à 0,45 $\mu m$. Six échantillons d'un volume de 100 ml sont prélevés et agités avec une vitesse de rotation de 300 tours par minute. A la température $T_1 = 35°C$ des masses de THK en poudre telles que 25, 50, 75, 100, 125 et 150 mg sont ajoutées et dissoutes dans les différents échantillons.

Après dissolution, la température est abaissée et maintenue à $T_2 = -5°C$ pendant une durée $H = 2$ heures, puis remontée à $T_m = +10°C$. A cette température $T_m$, chaque échantillon est miré. On note que seuls les échantillons à 25 et 50 mg sont limpides alors que les échantillons à 75, 100, 125 et 150 mg présentent un trouble. L'IST attribué à ce vin est donc 50/75.

Si une meilleure précision est nécessaire, il convient de recommencer le test en utilisant un gradient de charges de THK compris entre 50 et 75 mg.

Pour que la comparaison entre l'IST de plusieurs vins soit significative, il convient que les vins soient du même cru et surtout que les tests de mesure aient été effectués dans des conditions standard. Par exemple, pour un cru donné, un vin dont l'IST est 125/150 est plus stable qu'un vin ayant un IST de 50/75. Et dans les cas les plus favorables, cette comparaison suffit pour déterminer si un vin est stable ou instable. Par exemple, si l'IST d'un vin est supérieur à l'IST d'un vin stable, le vin testé est lui-même stable. Au contraire, si l'IST d'un vin est inférieur à l'IST d'un vin instable, le vin testé est lui-même instable.

Pour donner un sens plus concret à la notion d'IST, il est nécessaire de définir pour un cru ou une catégorie de vins, un Indice de Stabilité Tartrique Critique (ISTC) comme étant le seuil de discrimination entre les vins stables et les vins instables vis-à-vis d'une précipitation tartrique. Le détermination de l'ISTC est obtenue par étalonnage en couplant la méthode classique de conservation au froid et la méthode objet de la présente invention. Si on constate par exemple que seuls les vins ayant un IST inférieur ou égal à 50/75 précipitent durant un séjour de 6 jours à -5°C, l'ISTC du cru est 75.

S'il est utile au cours du stockage et de la conservation d'un vin de connaître l'évolution de son IST au même titre que les autres paramètres physicochimiques habituels, sa détermination au moment de la mise en bouteille revêt un intérêt particulier puisqu'elle fournit à l'utilisateur un critère objectif de décision.

En effet, compte tenu des définitions ci-dessus, deux cas seulement sont à envisager :
- IST $\leq$ ISTC, le vin est instable et un traitement de stabilisation est nécessaire.
- IST $>$ ISTC, le vin est naturellement stable et un traitement de stabilisation est inutile.

Il est à signaler que le test peut également être utilisé pour vérifier l'efficacité du traitement de stabilisation tartrique, ce traitement devant se traduire évidemment par une augmentation de l'IST.

Après description du "test de mesure" de l'IST adapté au laboratoire d'oenologie, on peut décrire le "test de décision" qui en est une version simplifiée plus adaptée à la cave. L'exemple suivant donné à titre indicatif et non limitatif illustre l'invention.

Le vin à tester appartient à un cru dont l'ISTC est 75. On prélève un seul échantillon de 100 ml de vin dans lequel on dissout par agitation à $T_1 = 35°C$ une masse de 75mg de THK. Après dissolution complète et toujours sous agitation, la température de l'échantillon est abaissée et maintenue à $T_2 = -5°C$ pendant une durée $H = 2$ heures. La température est ensuite remontée à $T_m = +10°C$. La présence d'un trouble indique que le traitement est indispensable, l'absence d'un trouble indique que le traitement est inutile.

Dans le procédé, objet de la présente invention, l'ajout de THK peut être remplacé par l'ajout de tartrates alcalins ou alcalino-terreux ou par l'ajout d'un mélange de THK et d'autres tartrates (à titre indicatif et non limitatif, le mélange peut être 95% de THK et 5% de tartrate de calcium.

## Revendications

1. Procédé pour évaluer le risque de précipitation d'un composé solide dans du vin, caractérisé par les étapes suivantes :
- on prélève $\underline{n}$ échantillons de vin,
- on porte ces échantillons à une température $(T_1)$ supérieure ou égale à la température ambiante et on ajoute à ceux-ci des masses différentes dudit composé solide, de façon à obtenir un gradient de concentration connu en ce composé,
- après dissolution du composé, la température des échantillons est abaissée jusqu'à une certaine valeur $(T_2)$ et est stabilisée à celle-ci,
- la température est ensuite remontée jusqu'à une valeur $(T_m)$ inférieure à $(T_1)$ à laquelle est observée la présence ou l'absence d'un trouble,
- on note la concentration à laquelle et au-dessous de laquelle on observe l'absence de trouble et la concentration à laquelle et au-dessus de laquelle on observe la présence d'un trouble et on caractérise l'indice de stabilité du vin testé au moyen de ces deux concentrations.

2. Procédé conforme à la revendication 1, destiné à l'évaluation du risque de précipitation d'hydrogénate de potassium ou de tartrate alcalin ou alcalino-terreux, caractérisé en ce qu'on ajoute aux échantillons de l'hydrogénate de potassium et/ou un tartrate alcalin ou alcalino-terreux.

3. Procédé selon la revendication 1, caractérisé en ce que le vin testé est préalablement filtré au moyen d'un filtre dont la porosité est comprise entre 0,1 μm et 100μm.

4. Procédé selon la revendication 1, caractérisé en ce que des masses connues de composé comprises entre 0 et 1,5 g par litre sont dissoutes dans le vin testé.

5. Procédé selon l'une des revendications 1 à 4, caractérisé en ce que la série d'échantillons est soumise à un traitement thermique comportant trois niveaux de température successifs $T_1$, $T_2$ et $T_m$ tels que $50°C > T_1 > T_m \geqq T_2$

> - 10°C, où $T_1$ est la température de dissolution du composé, $T_2$ la température à laquelle la série d'échantillons est maintenue pendant une durée comprise entre 0 et 10 heures et $T_m$ la température à laquelle est observé le résultat du test.

6. Procédé conforme à l'une des revendications 1 à 5, caractérisé en ce que les échantillons de vin sont en outre conservés au froid à - 5°C pendant 6 jours, on détermine un indice de stabilité critique (ISTC) du cru du vin en notant les concentrations en composé de l'échantillon pour lesquelles on observe une précipitation, on compare cet indice (ISTC) avec l'indice (IST) déterminé par le méthode précitée et on stabilise le vin seulement si l'indice (IST) est inférieur ou égale à l'indice (ISTC).